# EUROPEAN PATENT APPLICATION

(11) **EP 4 837 551 A1**
(43) Date of publication of application: **11.11.2026**
(21) Application number: 25777712.8
(22) Date of filing: 24.03.2025
(51) Int. Cl.: A61F 13/49, A61F 13/53, A61F 13/496

(54) **UNDERWEAR-STYLE ABSORBENT ARTICLE**

(30) Priority: 25.03.2024 JP 2024048787; 25.03.2024 JP 2024048788; 25.03.2024 JP 2024048789; 31.05.2024 JP 2024089431
(71) Applicant: Unicharm Corporation, Ehime 799-0111 (JP)
(72) Inventor: UTSUNOMIYA, Hideto, Kanonji-shi, Kagawa 769-1602 (JP); NARA, Yuki, Kanonji-shi, Kagawa 769-1602 (JP); MUKAI, Hirotomo, Kanonji-shi, Kagawa 769-1602 (JP)
(74) Representative: Staeger & Sperling Partnerschaftsgesellschaft mbB
(86) International application number: PCT/JP2025/011482
(87) International publication number: WO 2025/205622

(57) **Abstract**

An underwear-style absorbent article (1) comprises an absorbent body (10) and an exterior member (20) and is provided with a pair of leg opening parts (LHC) on both sides of the exterior member (20) in the left-right direction. The underwear-style absorbent article (1) includes a crotch elastic member (40) that is provided closer to a non-skin side than an absorbent core (11) and expands and contracts in the longitudinal direction, and the length (Lef) of the crotch elastic member (40) frontward of a central location (CL) in the longitudinal direction is longer than the length (Leb) of the crotch elastic member (40) rearward of the central location (CL). In the left-right direction, the entirety of a peripheral edge of each leg opening part (LHC) is located outward of an outer end (10es) of the absorbent body (10). The exterior member (20) does not include a leg-surrounding elastic member that extends across the central location (CL) in the longitudinal direction and expands and contracts along the peripheral edge of each leg opening part (LHC).

## Description

### FIELD

The present invention relates to an underpants-shaped absorbent article.

### BACKGROUND

In an underpants-shaped absorbent article (for example, an underpants-shaped diaper) including an absorbent main body and a waist member arranged so as to cover the front and back portions of the absorbent main body from the non-skin side, a technique has been known in which an elastic member (crotch elastic member) that stretches and contracts along the longitudinal direction of the absorbent main body is provided to improve fit in a crotch portion of the absorbent main body. For example, Patent Document 1 discloses a wearable article including a chassis 100 (exterior member) and an absorbent main body 200, the wearable article further including a central elastic member 41C (crotch elastic member) overlaid on a non-skin-side surface of an absorbent body and is arranged so as to extend along the longitudinal direction in a substantially central portion in a width direction.

### [CITATION LIST]

### [Patent Literature]

[Patent Document 1] Japanese Unexamined Patent Application Publication (Translation of PCT Application) No. 2011-132688

### SUMMARY

### [TECHNICAL PROBLEM]

When such an absorbent article including a crotch elastic member is worn, the absorbent main body having been deformed into a shape convex toward the skin side by an elastic force of the crotch elastic member can fit the crotch portion by being pulled upward together with the exterior member. However, depending on the arrangement of the crotch elastic member provided to the absorbent main body and the shape of leg openings formed in the exterior member, there may be cases where it is difficult to sufficiently improve fit in the crotch portion. For example, when the crotch elastic member is provided relatively on the back side in the longitudinal direction, the absorbent main body may not easily fit an excretory opening (a urethral opening, a vaginal opening, and/or the like). Further, when the leg openings (leg portions) extend inward beyond two side ends of the absorbent main body as in Patent Document 1, it may become difficult to sufficiently pull upward the two side portions of the absorbent main body when worn, which may result in deterioration of fit in the crotch portion. Further, when a stretchable member (a leg elastic member or the like) extending continuously along the contour of each of the leg openings is provided, the absorbent main body may not easily undergo convex deformation in an intended manner, thereby tending to deteriorate the fit.

The present invention has been made in view of such an issue as described above, and is directed to providing an underpants-shaped absorbent article with a good fit in a crotch portion.

### [SOLUTION TO PROBLEM]

A main aspect of the present invention to achieve the above objective is an underpants-shaped absorbent article having a longitudinal direction, a lateral direction, and a thickness direction that intersect each other in an unfolded and stretched state, the underpants-shaped absorbent article including: an absorbent main body including an absorbent core; an exterior member provided on a non-skin side with respect to the absorbent main body; and a crotch elastic member, the exterior member including, on two lateral sides thereof, a pair of leg portions obtained such that a width in the lateral direction at a central portion in the longitudinal direction is smaller than a width thereof in the lateral direction at two end portions in the longitudinal direction, the crotch elastic member being provided on the non-skin side with respect to the absorbent core, the crotch elastic member being configured to stretch and contract along the longitudinal direction, in the longitudinal direction, a length of the crotch elastic member on a front side with respect to a central position being greater than a length of the crotch elastic member on a back side with respect to the central position, in the lateral direction, an entire peripheral edge of each of the leg portions being located on an outer side with respect to a corresponding outer end of the absorbent main body, the exterior member does not include a leg elastic member extending across the central position in the longitudinal direction and configured to stretch and contract along the peripheral edge of each of the leg portions.

Other features of the present invention will become apparent from the description in the present specification and the accompanying drawings.

### [ADVANTAGEOUS EFFECT OF THE INVENTION]

According to the present invention, it is possible to provide an underpants-shaped absorbent article with a good fit in a crotch portion.

### BRIEF DESCRIPTION OF DRAWINGS

FIG. 1 is a front view of an underpants-shaped disposable diaper 1 when viewed from the front side.
FIG. 2 is a plan view of the underpants-shaped disposable diaper 1 in an unfolded and stretched state.
FIG. 3 is a schematic cross-sectional view of the underpants-shaped disposable diaper 1 taken along line C-C in FIG. 2.
FIG. 4A is a plan view of an absorbent main body 10 when viewed from the skin side. FIG. 4B is a schematic cross-sectional view of the absorbent main body 10 at a longitudinal central position CL.
FIGS. 5A and 5B are diagrams illustrating convex deformation of the absorbent main body 10.
FIGS. 6A and 6B are diagrams illustrating an operation performed when putting on an underpants-shaped diaper 5 of a comparative example.
FIGS. 7A and 7B are diagrams illustrating an operation performed when putting on the underpants-shaped diaper 1 according to the present embodiment.
FIG. 8 is a plan view for illustrating a specific configuration of the diaper 1.
FIG. 9 is a plan view illustrating an arrangement of a main-body joining portion 70 that joins the absorbent main body 10 and an exterior member 20.

### DESCRIPTION OF EMBODIMENTS

At least following matters will become apparent from the descriptions of the present specification and the accompanying drawings.

### (Aspect 1)

An underpants-shaped absorbent article having a longitudinal direction, a lateral direction, and a thickness direction that intersect each other in an unfolded and stretched state, the underpants-shaped absorbent article including: an absorbent main body including an absorbent core; an exterior member provided on a non-skin side with respect to the absorbent main body; and a crotch elastic member, the exterior member including, on two lateral sides thereof, a pair of leg portions obtained such that a width in the lateral direction at a central portion in the longitudinal direction is smaller than a width thereof in the lateral direction at two end portions in the longitudinal direction, the crotch elastic member being provided on the non-skin side with respect to the absorbent core, the crotch elastic member being configured to stretch and contract along the longitudinal direction, in the longitudinal direction, a length of the crotch elastic member on a front side with respect to a central position being greater than a length of the crotch elastic member on a back side with respect to the central position, in the lateral direction, an entire peripheral edge of each of the leg portions being located on an outer side with respect to a corresponding outer end of the absorbent main body, the exterior member does not include a leg elastic member extending across the central position in the longitudinal direction and configured to stretch and contract along the peripheral edge of each of the leg portions.

According to the underpants-shaped absorbent article of Aspect 1, the crotch elastic member is provided relatively on the front side in the longitudinal direction, and thus the absorbent main body undergoes convex deformation toward the skin side in the vicinity of the wearer's excretory opening, thereby improving fit in the crotch portion. In this event, as a central portion of the absorbent main body in the lateral direction undergoes convex deformation toward the skin side, a gap may be likely to be formed between the wearer's groin region and the two lateral side portions of the absorbent main body. However, the peripheral edge of each of the leg portions forming the leg openings in the exterior member is located on the outer side with respect to the corresponding outer end of the absorbent main body, and thus the two side portions of the absorbent main body are easily pulled upward by the exterior member when the exterior member is pulled upward. Accordingly, the convexly deformed absorbent main body becomes likely to fit the crotch portion, and formation of gaps between the wearer's groin region and the two side portions of the absorbent main body is suppressed. Further, since a leg elastic member extending continuously along each of the leg portions of the exterior member is not provided, excessive upward pulling caused by the elastic force of the leg elastic member is suppressed when the exterior member is pulled upward. Accordingly, digging of the two side portions of the absorbent main body and the leg portions into the wearer's groin region and hindrance of convex deformation of the absorbent main body are suppressed. This can improve fit in the crotch portion of the underpants-shaped absorbent article.

### (Aspect 2)

The underpants-shaped absorbent article according to Aspect 1, wherein the exterior member includes a first sheet extending continuously from one side to another side in the longitudinal direction, and second sheets laminated to the first sheet at two end portions in the longitudinal direction, respectively, the second sheets not being laminated to the first sheet at a central portion in the longitudinal direction.

According to the underpants-shaped absorbent article of Aspect 2, in the vicinity of the two end portions in the longitudinal direction, strength can be ensured by configuring the waist member as a structure having at least two layers, thereby suppressing tearing when the exterior member is grasped and pulled upward. On the other hand, in the vicinity of a central portion in the longitudinal direction that comes into contact with the wearer's crotch portion, the number of layers of the exterior member is reduced, which causes the stiffness of the exterior member to be lower than that at the two end portions, and thus flexibility in the crotch portion is more likely to be ensured. Accordingly, the convexly deformed absorbent main body can be more likely to fit the wearer's crotch portion.

### (Aspect 3)

The underpants-shaped absorbent article according to Aspect 1 or 2, wherein an area of a portion of the exterior member on a front side with respect to the central position in the longitudinal direction, the portion being where the second sheet is not laminated to the first sheet, is greater than an area of a portion of the exterior member on the back side with respect to the central position in the longitudinal direction, the portion being where the second sheet is not laminated to the first sheet.

According to the underpants-shaped absorbent article of Aspect 3, in a region of the crotch portion on the front side, a portion having a smaller number of layers of the exterior member is larger than in a region on the back side, and thus flexibility can be more likely to be secured on the front side in the crotch portion. Accordingly, during wearing, the exterior member is more likely to be flexibly deformed in a region that comes into contact with the wearer's excretory opening, thereby making it less likely to cause discomfort to the wearer. Further, convex deformation of the absorbent main body is less likely to be hindered in the vicinity of the excretory opening, thereby being able to improve the fit.

### (Aspect 4)

The underpants-shaped absorbent article according to any one of Aspects 1 to 3, wherein in the exterior member, a lateral elastic member arranged linearly along the lateral direction is provided between the first sheet and each of the second sheets in the thickness direction, and a shortest distance from the central position in the longitudinal direction to the lateral elastic member on the front side with respect to the central position is greater than a shortest distance from the central position to the lateral elastic member on the back side with respect to the central position.

According to the underpants-shaped absorbent article of Aspect 4, in the longitudinal direction, as the distance from the central position to a position at which the lateral elastic member is provided becomes greater, the area of a region in which stiffness is low can be increased. Accordingly, by making the distance from the central position to the lateral elastic member on the front side greater than the distance from the central position to the lateral elastic member on the back side, the area of such low-stiffness region of the exterior member on the front side in the crotch portion is increased. Accordingly, flexibility of the exterior member can be more likely to be secured in the vicinity of the wearer's excretory opening. Further, convex deformation of the absorbent main body is less likely to be hindered in the vicinity of the excretory opening, thereby being able to improve the fit.

### (Aspect 5)

The underpants-shaped absorbent article according to any one of Aspects 1 to 4, wherein the exterior member does not include an elastic member arranged obliquely with respect to the longitudinal direction.

According to the underpants-shaped absorbent article of Aspect 5, when the exterior member is pulled upward during wearing, an excessively large force is less likely to act in an oblique direction, thereby suppressing collapse of the shape of the convexly deformed absorbent main body. Accordingly, deterioration of the fit is likely to be suppressed.

### (Aspect 6)

The underpants-shaped absorbent article according to any one of Aspects 1 to 5, wherein the absorbent core has, at a central portion in the longitudinal direction, a constricted portion having a width in the lateral direction smaller than a width at two end portions in the longitudinal direction, and in the lateral direction, a maximum length between the outer end of the absorbent main body and a corresponding peripheral edge of the constricted portion is greater than a minimum length between the outer end of the absorbent main body and a corresponding peripheral edge of the leg portion.

According to the underpants-shaped absorbent article of Aspect 6, by making the constriction of the absorbent core in the lateral direction as large as possible, the edge portions of the absorbent core are less likely to interfere with the wearer's legs or groin region, thereby being able to make it less likely for the wearer to feel the hardness of the absorbent core. On the other hand, by making the length of portions of the exterior member protruding outward beyond the absorbent main body in the lateral direction as small as possible, the area of direct contact between the exterior member and the wearer's skin is reduced, thereby being able to make it less likely to cause discomfort.

### (Aspect 7)

The underpants-shaped absorbent article according to any one of Aspects 1 to 6, wherein a portion of the exterior member having a smallest width in the lateral direction is located on the front side with respect to the central position in the longitudinal direction.

According to the underpants-shaped absorbent article of Aspect 7, since the width of the exterior member is smallest at a portion relatively on the front side in the crotch portion, the edge portions of the exterior member (peripheral edges of the leg portions) are less likely to interfere with movements of the wearer's legs when the wearer moves his or her leg(s) forward while wearing the underpants-shaped absorbent article. This can reduce discomfort in the crotch portion.

### (Aspect 8)

The underpants-shaped absorbent article according to any one of Aspects 1 to 7, wherein the portion of the exterior member having the smallest width in the lateral direction is located in a region overlapping the crotch elastic member in the longitudinal direction.

According to the underpants-shaped absorbent article of Aspect 8, by minimizing the area of the exterior member on two lateral sides of a region in which the elastic force of the crotch elastic member acts, resistance generated when the absorbent main body undergoes convex deformation is reduced, thereby facilitating deformation of the absorbent main body into a convex shape. This can further improve the fit of the absorbent main body in the crotch portion.

### (Aspect 9)

The underpants-shaped absorbent article according to any one of Aspects 1 to 8, wherein a main-body joining portion that joins the absorbent main body and the exterior member is provided on two sides in the lateral direction of a region overlapping the crotch elastic member in the longitudinal direction.

According to the underpants-shaped absorbent article of Aspect 9, since the absorbent main body and the exterior member are firmly joined and integrated by the main-body joining portion on two lateral sides of the crotch elastic member, lifting of the two side portions of the absorbent main body away from the exterior member is suppressed. Accordingly, discomfort in the vicinity of the wearer's groin region during wearing can be reduced.

### (Aspect 10)

The underpants-shaped absorbent article according to any one of Aspects 1 to 9, wherein a width of the main-body joining portion in the lateral direction in a region overlapping the crotch elastic member in the longitudinal direction is equal to a width of the main-body joining portion in the lateral direction at two end portions of the absorbent main body in the longitudinal direction.

According to the underpants-shaped absorbent article of Aspect 10, since the absorbent main body and the exterior member are joined together entirely in a substantially uniform manner, stress concentration and the like are less likely to occur when the exterior member is pulled upward when the underpants-shaped absorbent article is worn, thereby suppressing application of a localized force to the absorbent main body via the exterior member. Accordingly, convex deformation of the absorbent main body is less likely to be hindered, thereby being able to facilitate improvement of the fit in the crotch portion.

### (Aspect 11)

The underpants-shaped absorbent article according to any one of Aspects 1 to 10, wherein the absorbent main body does not include, at two end portions in the lateral direction, a leg elastic member configured to stretch and contract along the longitudinal direction.

According to the underpants-shaped absorbent article of Aspect 11, in a central portion in the lateral direction, the influence of the elastic force of the crotch elastic member is increased, and thus the absorbent core is more likely to be pushed toward the skin side and the absorbent main body more easily undergoes convex deformation.

This improves the fit in the crotch portion.

### (Aspect 12)

The underpants-shaped absorbent article according to any one of Aspects 1 to 11, wherein the exterior member does not include, in a region overlapping the crotch elastic member in the longitudinal direction, an elastic member configured to stretch and contract in the lateral direction.

According to the underpants-shaped absorbent article of Aspect 12, an elastic member configured to stretch and contract along the lateral direction is not provided in a region overlapping the crotch elastic member in the longitudinal direction, and thus stretching and contraction of the crotch elastic member along the longitudinal direction is less likely to be interfered with. Accordingly, the absorbent main body can more easily undergo convex deformation in an intended manner. This improves the fit in the crotch portion.

### (Aspect 13)

The underpants-shaped absorbent article according to any one of Aspects 1 to 12, wherein the exterior member includes, at two lateral end portions, a pair of side joining portions that join one-side end portions and another-side end portions in the longitudinal direction to each other, respectively, and in the unfolded and stretched state, a straight line connecting, by a shortest distance, a central position between a one-side end and another-side end in the longitudinal direction of each of the side joining portions provided on one side in the longitudinal direction and an end of the crotch elastic member on the one side in the longitudinal direction does not intersect a corresponding peripheral edge of the leg portion, and a straight line connecting, by a shortest distance, a central position between a one-side end and another-side end in the longitudinal direction of each of the side joining portions provided on the other side in the longitudinal direction and an end of the crotch elastic member on the other side in the longitudinal direction does not intersect the corresponding peripheral edge of the leg portion.

According to the underpants-shaped absorbent article of Aspect 13, when the two sides of the exterior member are grasped and pulled upward when wearing, a straight line connecting, by a shortest distance, a position at which the exterior member is grasped (the central position of each of the side joining portions) and the crotch elastic member is not interrupted by the corresponding leg portion, and thus, a force of pulling up the underpants-shaped absorbent article is easily transmitted along the straight line. Accordingly, the absorbent main body can be efficiently pulled upward via the exterior member. This enables the absorbent main body to be more easily fitted to the wearer's crotch portion.

### Embodiments

Hereinafter, an underpants-shaped disposable diaper 1 (hereinafter, also referred to as "diaper 1") will be described as an example of an underpants-shaped absorbent article according to the present invention. However, the underpants-shaped absorbent article according to the present invention is not limited to the above and may also be used as a sanitary napkin, sanitary shorts, and the like.

### <Basic configuration of underpants-shaped disposable diaper 1>

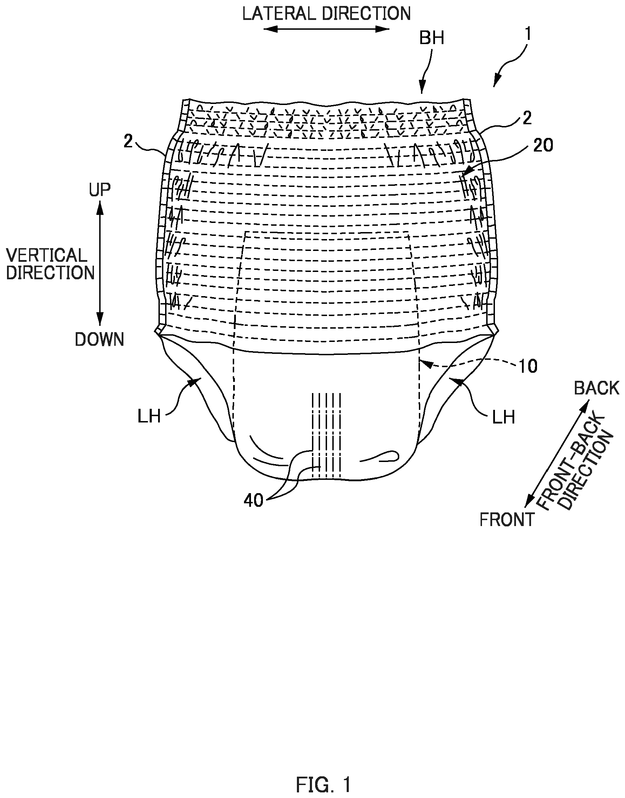

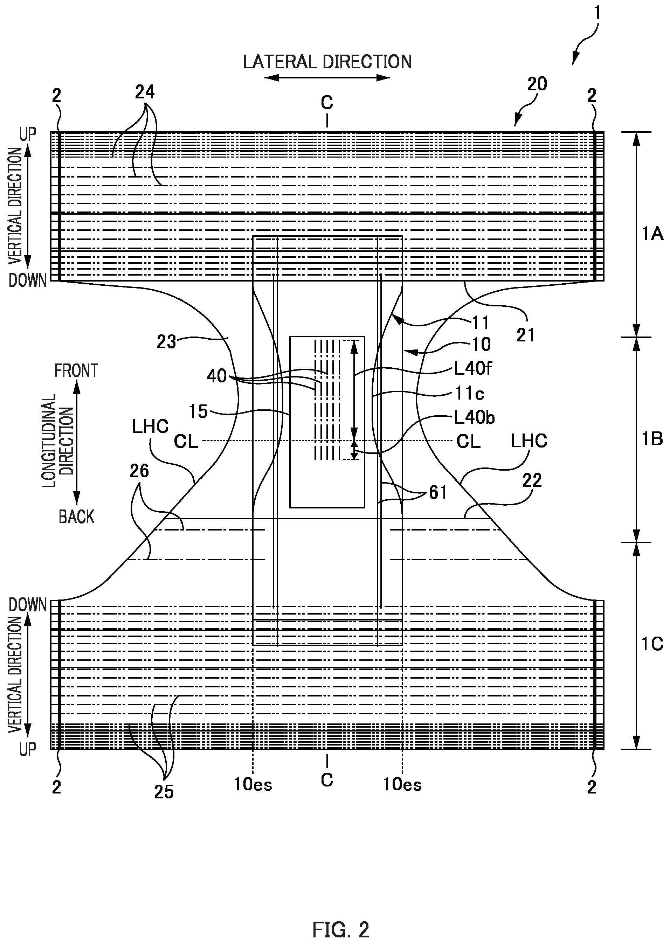

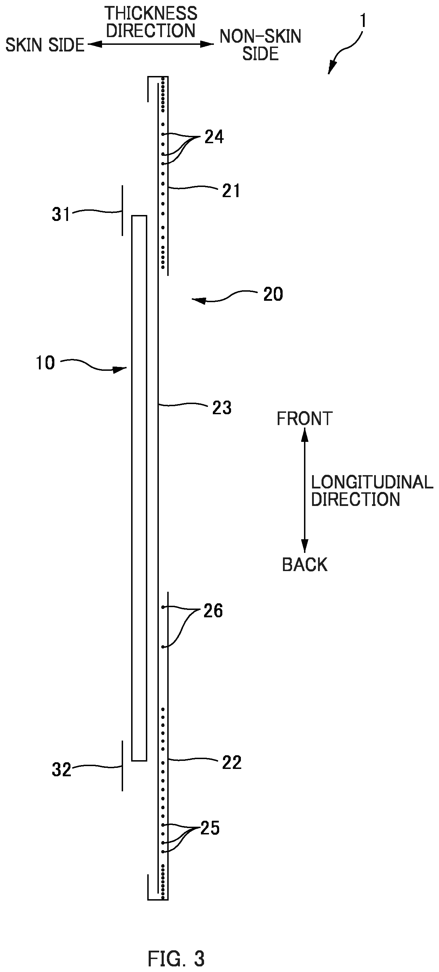

FIG. 1 is a front view of the underpants-shaped disposable diaper 1 when viewed from the front side. FIG. 2 is a plan view of the underpants-shaped disposable diaper 1 in an unfolded and stretched state. FIG. 3 is a schematic cross-sectional view of the underpants-shaped disposable diaper 1 taken along line C-C in FIG. 2.

The unfolded state of the underpants-shaped disposable diaper 1 (diaper 1) refers to a state in which a pair of side joining portions 2 provided at two side portions of the diaper 1 are pulled apart, and the diaper 1 is opened and laid out flat. The stretched state of the diaper 1 refers to a state in which the diaper 1 is stretched to the extent that wrinkles in the diaper 1 are no longer visible. Specifically, the stretched state is a state in which the diaper 1 is stretched until the dimensions of the members that constitute the diaper 1 (for example, a top sheet 12, an inner-layer sheet 23, and the like, described later) match or are close to the dimensions of the members on their own.

As shown in FIG. 1, the diaper 1 has a vertical direction, a lateral direction, and a front-back direction (the direction penetrating the plane of FIG. 1), which are orthogonal to one another, and includes a waist opening BH and a pair of leg openings LH. The upper side in the vertical direction corresponds to the side of the waist opening BH, and the lower side corresponds to the crotch side. The front side in the front-back direction corresponds to the front side of a wearer, and the back side corresponds to the back side of the wearer.

Further, in the unfolded state shown in FIG. 2, the diaper 1 has a longitudinal direction, a lateral direction, and a thickness direction (the direction penetrating the plane of FIG. 2), which intersect one another. The lateral direction in the unfolded state shown in FIG. 2 is the same as the lateral direction in the underpants-shaped state shown in FIG. 1. The longitudinal direction in the unfolded state corresponds to the direction along the vertical direction in the underpants-shaped state. One side in the longitudinal direction corresponds to the front (abdominal) side, and the other side corresponds to the back (back) side. Further, as shown in FIG. 3, the direction in which the materials constituting the diaper 1 are overlaid is defined as the thickness direction. In the thickness direction, the side that comes into contact with the skin of the wearer is referred to as a skin side, and the opposite side is referred to as a non-skin side.

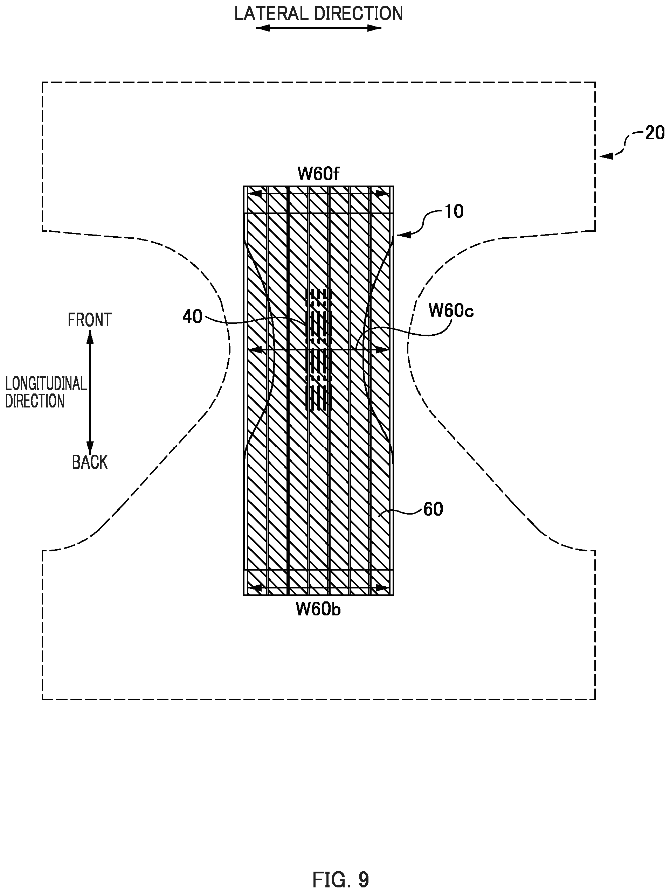

The diaper 1 includes an absorbent main body 10 and an exterior member 20 provided on the non-skin side with respect to the absorbent main body 10. The absorbent main body 10 and the exterior member 20 are joined together in the thickness direction via a main-body joining portion 70 (see FIG. 9). Cover sheets 31 and 32 that cover two longitudinal end portions of the absorbent main body 10 from the skin side may also be provided (see FIG. 3). This can suppress the edges of the absorbent main body 10 from digging into the wearer and can also suppress opening of the absorbent main body 10.

In the present specification, as shown in FIG. 2, in the unfolded and stretched state of the diaper 1, the front-side region obtained when the diaper 1 is divided into three equal parts in the longitudinal direction is referred to as "front portion 1A," the central region is referred to as "crotch portion 1B," and the back-side region is referred to as "back portion 1C." From the unfolded state shown in FIG. 2, the diaper 1 is folded in two at a central position CL in the longitudinal direction, and portions of the front portion 1A and the back portion 1C that face each other in the thickness direction (front-back direction) are connected at the two lateral side portions by the pair of side joining portions 2, thereby forming the underpants-shaped diaper 1 as shown in FIG. 1. Examples of a joining method for the side joining portions 2 include heat sealing, ultrasonic sealing, adhesive bonding, and the like.

### (Absorbent main body 10)

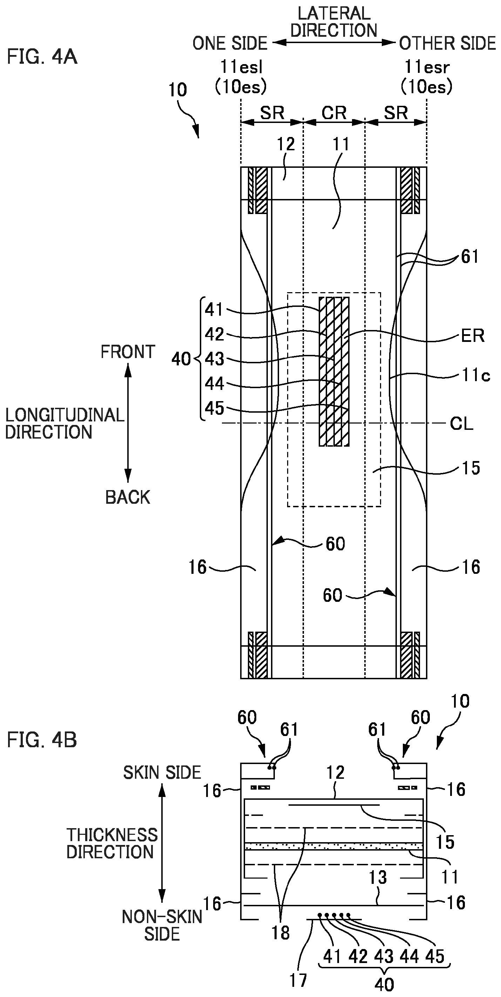

FIG. 4A is a plan view of the absorbent main body 10 when viewed from the skin side. FIG. 4B is a schematic cross-sectional view of the absorbent main body 10 taken along the central position CL in the longitudinal direction. The absorbent main body 10 includes: an absorbent core 11 that absorbs and retains excreted fluid such as urine; a liquid-permeable top sheet 12 (for example, a nonwoven fabric) arranged on the skin side with respect to the absorbent core 11; a liquid-impermeable back sheet 13 (for example, a resin film made of polyethylene, polypropylene, or the like) arranged on the non-skin side with respect to the absorbent core 11; a liquid-permeable intermediate sheet 15 (for example, a nonwoven fabric) arranged between the top sheet 12 and the absorbent core 11; a pair of side sheets 16; and an elastic-member cover sheet 17.

The absorbent core 11 absorbs and retains excreted fluid such as urine. The surface of the absorbent core 11 may be covered with a liquid-permeable core-wrapping sheet 18 (for example, tissue paper). The absorbent core 11 is formed by shaping a liquid absorbent material, such as water-retentive fibers and a superabsorbent polymer (so-called SAP), into a predetermined shape. In the present embodiment, as shown in FIG. 4A, the absorbent core 11 has a substantially hourglass shape in which a longitudinal central portion is constricted inward in the lateral direction. However, the planar shape of the absorbent core 11 is not particularly limited, and for example, the absorbent core 11 may be formed in a rectangular shape in plan view. In the present embodiment, the width (maximum width) of the absorbent main body 10 in the lateral direction is substantially equal to the width (maximum width) of the absorbent core 11 in the lateral direction.

A pair of leak-proof wall portions 60 extending along the longitudinal direction are provided to two lateral side portions of the absorbent main body 10. As shown in FIG. 4B, portions of the side sheets 16 extending from two lateral side portions of the non-skin-side surface of the back sheet 13 are folded at a plurality of locations and are provided at two lateral side portions on the skin-side surface of the top sheet 12, thereby forming the leak-proof wall portions 60. Leak-proof-wall elastic members 61 (for example, elastic strings) are attached to the leak-proof wall portions 60 in a stretched state along the longitudinal direction. When the diaper 1 is worn, the leak-proof wall portions 60 stand up toward the skin side of the wearer due to the elasticity exhibited by the leak-proof-wall elastic members 61, thereby making it possible to block excrement.

The absorbent main body 10 further includes a crotch elastic member 40 configured to stretch and contract in the longitudinal direction in a lateral central portion and on the non-skin side with respect to the absorbent core 11 in the thickness direction. Specifically, the crotch elastic member 40 in the present embodiment is attached, in a stretched state along the longitudinal direction, between the back sheet 13 provided on the non-skin side with respect to the absorbent core 11 and the elastic-member cover sheet 17 arranged on the non-skin side with respect to the back sheet 13 in the thickness direction. However, the crotch elastic member 40 only needs to be provided on the non-skin side with respect to the absorbent core 11. For example, the crotch elastic member 40 may be provided between the core-wrapping sheet 18 and the back sheet 13 on the non-skin side with respect to the absorbent core 11.

In FIG. 4A, five crotch elastic members 41 to 45 are arranged in the lateral central portion of the absorbent main body 10 (absorbent core 11) so as to extend along the longitudinal direction and be spaced apart from each another in the lateral direction. Hereinafter, a region defined by the five crotch elastic members 40 (41 to 45) is also referred to as "crotch elastic region ER." That is, as indicated by a hatched portion in FIG. 4A, a region surrounded by the crotch elastic member 41 arranged farthest on one side in the lateral direction and the crotch elastic member 45 arranged farthest on the other side in the lateral direction constitutes the crotch elastic region ER. However, the number and arrangement of the crotch elastic members 40 are not limited thereto.

Further, a region located at the center when the absorbent core 11 (absorbent main body 10) is equally divided into three portions in the lateral direction is defined as a "central region CR," and regions located on two sides of the central region CR are defined as "two side regions SR." Here, "equally divided into three portions" means that the distance (width) between one lateral end 11esl and the other lateral end 11esr of the absorbent core 11 is divided into three equal portions in FIG. 4A. As shown in FIG. 4A, in the diaper 1, the crotch elastic region ER is provided in the central region CR. Specifically, at least one of the crotch elastic members 41 to 45 forming the crotch elastic region ER is provided so as to overlap the central region CR when viewed in the thickness direction. When the diaper 1 is worn, the central region CR of the absorbent core 11 is three-dimensionally deformed so as to protrude toward the skin side due to the elasticity exhibited by the crotch elastic member 40 (crotch elastic region ER), thereby being able to fit with respect to the crotch portion and the excretory opening, which makes it possible to achieve good absorption performance and fit.

### (Exterior member 20)

The exterior member 20 is a sheet member having a shape in which a longitudinal central portion is constricted inward in the lateral direction. As shown in FIGS. 2 and 3, the exterior member 20 includes a front outer-layer sheet 21, a back outer-layer sheet 22, the inner-layer sheet 23, waist elastic members 24 and 25, and buttocks elastic members 26.

Examples of the front outer-layer sheet 21, the back outer-layer sheet 22, and the inner-layer sheet 23 include flexible sheet members such as nonwoven fabrics or the like. The inner-layer sheet 23 forms the outer shape of the exterior member 20 and is a continuous sheet member extending from the front portion 1A to the back portion 1C via the crotch portion 1B. The front outer-layer sheet 21 is arranged on the non-skin side with respect to the inner-layer sheet 23 and in a portion of the front portion 1A. The front outer-layer sheet 21 overlaps the side joining portions 2 in the longitudinal direction and has the same length in the longitudinal direction as that of the side joining portions 2. The back outer-layer sheet 22 is arranged on the non-skin side with respect to the inner-layer sheet 23 and in the back portion 1C and in a portion of the crotch portion 1B. The back outer-layer sheet 22 overlaps the side joining portions 2 in the longitudinal direction and extends toward the crotch side beyond the side joining portions 2. In the diaper 1 in the unfolded state, the front outer-layer sheet 21 and the back outer-layer sheet 22 are spaced apart from each other in the longitudinal direction, and the inner-layer sheet 23 is exposed to the outside as the non-skin-side surface of the diaper 1 therebetween.

In FIG. 3, a portion of the front outer-layer sheet 21 (back outer-layer sheet 22) is folded back toward the abdominal side in the thickness direction at the front end (back end) of the exterior member 20 in the longitudinal direction, however, the front outer-layer sheet 21 (back outer-layer sheet 22) is not necessarily folded back. A portion of the inner-layer sheet 23 may be folded back at the front end (back end) of the exterior member 20 in the longitudinal direction.

The waist elastic members 24 and 25 and the buttocks elastic members 26 are elastic members configured to stretch and contract in the lateral direction, and examples thereof include elastic strings. As shown in FIG. 2, the waist elastic members 24 and 25 overlap the side joining portions 2 in the longitudinal direction. In the following description, the waist elastic member located in the front portion 1A is also referred to as front waist elastic member 24, and the waist elastic member 25 located in the back portion 1C is also referred to as back waist elastic member 25. The buttocks elastic members 26 are located on the crotch side in the longitudinal direction (on the lower side in the vertical direction) with respect to the side joining portions 2. Although the number of buttocks elastic members 26 in the present embodiment is two, the number thereof is not particularly limited thereto.

The waist elastic members 24 and 25 and the buttocks elastic members 26 are arranged side by side in the vertical direction in the underpants-shaped state between the inner-layer sheet 23 and the front outer-layer sheet 21 or the back outer-layer sheet 22, and are fixed in a stretched state along the lateral direction. By virtue of the elastic force in the lateral direction generated by the waist elastic members 24 and 25 and the buttocks elastic members 26, the exterior member 20 fits around the waist and to the buttocks of the wearer.

In the exterior member 20 in the unfolded state shown in FIG. 2, portions that are constricted in the lateral direction at the central portion in the longitudinal direction forms the pair of leg openings LH when the diaper 1 is in the underpants-shaped state as shown in FIG. 1. The exterior member 20 is cut to form such constricted portions such that the width thereof in the lateral direction at the central portion in the longitudinal direction is smaller than the width thereof in the lateral direction at two end portions in the longitudinal direction. Hereinafter, this constricted portion having the reduced width in the lateral direction is also referred to as "leg portions LHC." In the present embodiment, the leg portions LHC are each defined by a continuous curved line extending from the front portion 1A to the back portion 1C in the longitudinal direction and is formed such that, as shown in FIG. 2, the width of the exterior member 20 in the lateral direction is smallest at a position on the front side with respect to the central position CL in the longitudinal direction. Accordingly, when the wearer moves his or her leg(s) forward while wearing the underpants-shaped diaper 1 (for example, while walking), the leg portions LHC are less likely to interfere with movements of the legs.

### <Fit of absorbent main body 10>

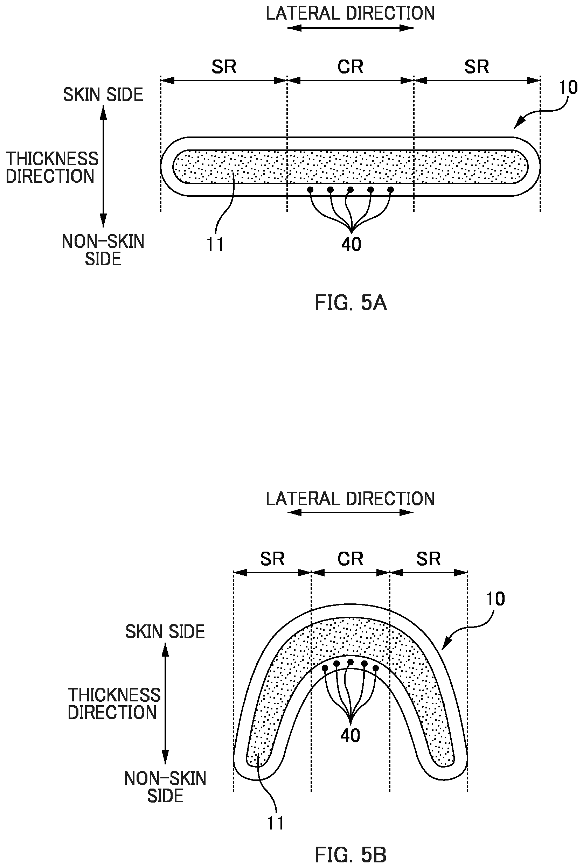

As described above, in the diaper 1 of the present embodiment, the absorbent main body 10 undergoes convex deformation toward the skin side when the diaper 1 is worn, thereby improving the fit in the wearer's crotch portion. FIGS. 5A and 5B are diagrams illustrating convex deformation of the absorbent main body 10. FIG. 5A is a schematic cross-sectional view illustrating the absorbent main body 10 in a stretched state, and FIG. 5B is a schematic cross-sectional view illustrating the absorbent main body 10 in a convexly deformed state.

Before the diaper 1 is worn, the absorbent main body 10 has a substantially planar shape as shown in FIG. 5A. In this state, the crotch elastic member 40 (crotch elastic region ER) in a state of being stretched in the longitudinal direction is provided on the non-skin side with respect to the absorbent core 11 in the thickness direction and in a central portion (central region CR) in the lateral direction. When the diaper 1 is worn, the crotch elastic member 40 (crotch elastic region ER) that has been released from the stretched state contracts in the longitudinal direction, thereby causing a force of pushing the central region CR of the absorbent core 11 from the non-skin side toward the skin side as shown in FIG. 5B to act. On the other hand, in the two side regions SR of the absorbent core 11, the contracting force caused by the elastic member does not act thereon, and thus the force of pushing the two side regions SR toward the skin side does not act. As a result, the central portion (central region CR) of the absorbent core 11 (absorbent main body 10) in the lateral direction is pushed toward the skin side, which causes the absorbent core 11 to be deformed into a convex shape in cross section. Accordingly, in the crotch portion of the wearer, the convexly deformed absorbent main body 10 can more easily fit the wearer's excretory opening (such as a urethral opening, a vaginal opening, and/or the like).

When the underpants-shaped diaper 1 including the crotch elastic member 40 and the absorbent main body 10 having the central region CR capable of convex deformation is put on, the wearer's legs are first inserted through the pair of leg openings LH, and then the exterior member 20 is pulled upward so that the absorbent main body 10 is pulled upward to the crotch portion and fitted thereto. In this event, depending on the shape of the exterior member 20, specifically, the shape of the leg openings LH (leg portions LHC), the absorbent main body 10 may not be pulled upward in an intended manner, resulting in deterioration of the fit.

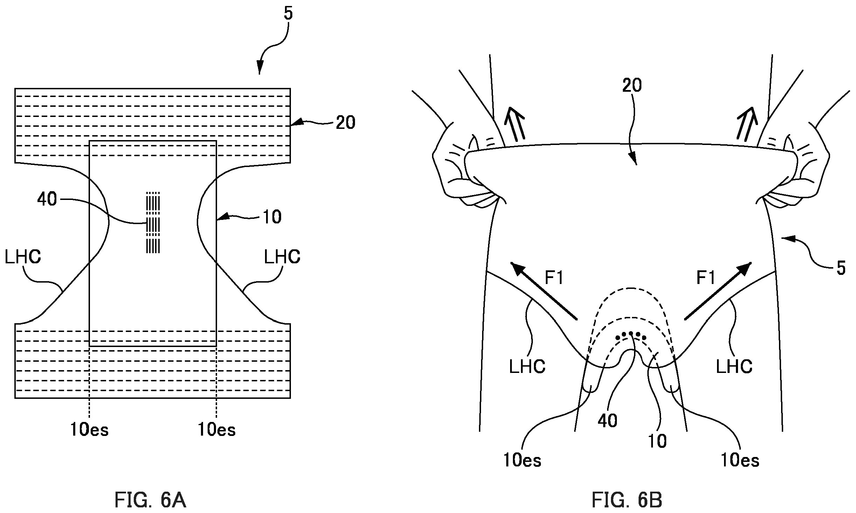

FIGS. 6A and 6B are diagrams illustrating an operation performed when putting on an underpants-shaped diaper 5 of a comparative example. FIG. 6A is a schematic plan view of the underpants-shaped diaper 5 in an unfolded and stretched state, and FIG. 6B illustrates an operation of wearing the underpants-shaped diaper 5. The underpants-shaped diaper 5 of the comparative example (hereinafter also referred to as "diaper 5") has substantially the same configuration as the diaper 1 of the present embodiment, and includes the absorbent main body 10 and the exterior member 20. The crotch elastic member 40 is provided on the non-skin side of the absorbent main body 10. On the other hand, as shown in FIG. 6A, in the diaper 5, a portion of the leg portion LHC overlaps the absorbent main body 10. In other words, in the lateral direction, each of the innermost ends of the leg portions LHC (that is, the portion of the exterior member 20 corresponding to the smallest width thereof) is located on the inner side with respect to an outer end 10es of the absorbent main body 10. The remaining configuration is the same as that of the diaper 1.

When the diaper 5 is put on, if a user grasps the two side portions of the exterior member 20 and pulls them upward, tensions as indicated by arrows F1 in FIG. 6B act along the peripheral edges of the leg portions LHC, thereby generating a force of pulling the absorbent main body 10 obliquely upward. At this time, in the diaper 5, each of the inner ends of the leg portions LHC is located on the inner side with respect to the outer end 10es of the absorbent main body 10 in the lateral direction, and thus two lateral end portions of the absorbent main body 10 protrude outward beyond the leg portion LHC, as shown in FIG. 6B, which makes it difficult for the upward pulling force to be effectively transmitted thereto. Accordingly, the absorbent main body 10 is less likely to be sufficiently pulled upward to the wearer's crotch portion, and the convexly deformed absorbent main body 10 is less likely to fit the wearer's excretory opening. Further, the two side regions SR of the absorbent main body 10 tend to become spaced apart from the wearer's groin region thereby creating gaps and becoming susceptible to positional displacement, and thus side leakage of excreta and discomfort to the wearer are more likely to occur.

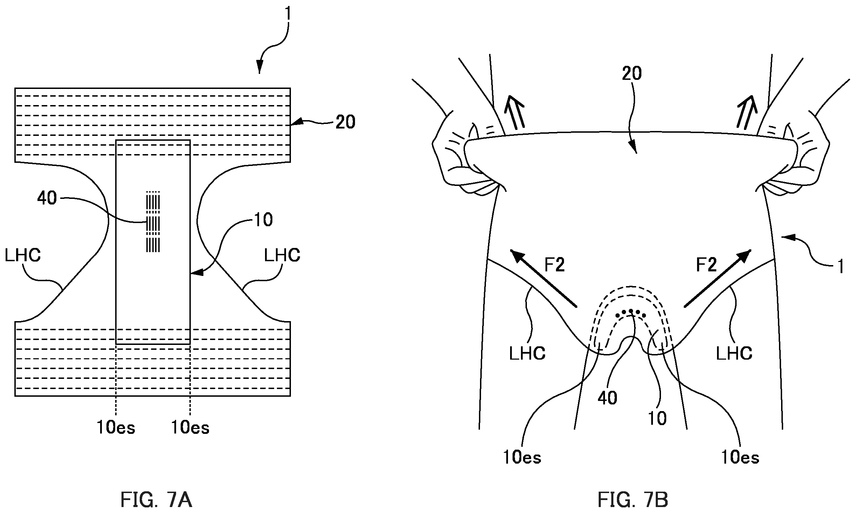

In contrast, in the diaper 1 according to the present embodiment, the fit of the absorbent main body 10 in the crotch portion during wearing is improved by adjusting the positional relationship between the absorbent main body 10 and the leg portions LHC of the exterior member 20. FIGS. 7A and 7B are diagrams illustrating an operation performed when putting on the underpants-shaped diaper 1 according to the present embodiment. FIG. 7A is a schematic plan view of the diaper 1 in an unfolded and stretched state, and FIG. 7B is a diagram illustrating an operation of putting on the diaper 1.

When the diaper 1 is put on, if a user grasps the two side portions of the exterior member 20 and pulls them upward, tensions as indicated by arrows F2 in FIG. 7B act along the peripheral edges of the leg portions LHC, thereby generating a force of pulling the absorbent main body 10 obliquely upward, as in the comparative example. In the diaper 1, the entirety of each of the leg portions LHC is located on the outer side with respect to the corresponding outer end 10es of the absorbent main body 10 in the lateral direction, and thus, as shown in FIG. 7B, the two lateral end portions of the absorbent main body 10 are easily pulled upward by the exterior member 20. Accordingly, the entire absorbent main body 10 becomes more likely to be pulled to the wearer's skin side and becomes more likely to fit the crotch portion. Further, gaps are less likely to be formed between the wearer's groin region and the two side regions SR of the absorbent main body 10, and thus side leakage of excreta and the like are also less likely to occur.

Furthermore, the exterior member 20 of the diaper 1 is not provided with an elastic member extending across the central position CL in the longitudinal direction, the elastic member being configured to stretch and contract along the peripheral edges of the leg portions LHC. For example, if an elastic member such as a leg-gather elastic member continuously extending so as to border the peripheral edge of each of the leg portions LHC of the exterior member 20 were provided, an elastic force would be more likely to act along the peripheral edge of each of the leg portions LHC due to the elastic member. In this case, in FIG. 7B, a force of pulling upward the two lateral end portions of the absorbent main body 10 (the force indicated by arrows F2) may act excessively strongly, which may cause discomfort in the wearer's groin region or cause collapse of the convex deformation of the absorbent main body 10 (absorbent core 11).

In contrast, in the diaper 1, when the exterior member 20 is pulled upward, the elastic force does not act along the peripheral edges of the leg portions LHC, and thus the two lateral end portions of the absorbent main body 10 are restrained from being pulled upward excessively strongly. Accordingly, the two end portions of the absorbent main body 10 and the leg portions LHC are restrained from digging into the wearer's groin region and the convex deformation of the absorbent main body 10 is restrained from collapsing.

Further, as shown in FIG. 2, in the longitudinal direction of the diaper 1, a length L40f of the crotch elastic member 40 on the front side with respect to the central position CL is greater than a length L40b of the crotch elastic member 40 on the back side with respect to the central position CL (L40f > L40b). That is, in the absorbent main body 10 (absorbent core 11) of the diaper 1, the elastic force of the crotch elastic member 40 is more likely to act on the front side than on the back side in the front-back direction. Thus, convex deformation of the absorbent main body 10 as shown in FIG. 5B is more likely to occur on the front side in the longitudinal direction, and when the diaper 1 is worn, the central region CR of the absorbent main body 10 having been convexly deformed toward the skin side is more likely to fit the wearer's urethral opening and vaginal opening.

In the diaper 1 according to the present embodiment, with such a configuration, it is possible to improve the fit in the wearer's crotch portion during wearing.

Further, the exterior member 20 of the diaper 1 includes the inner-layer sheet 23 (corresponding to a first sheet) extending continuously from an end on one side (for example, the front side) to an end on the other side (for example, the back side) in the longitudinal direction, and the front outer-layer sheet 21 and the back outer-layer sheet 22 (each corresponding to a second sheet) laminated on the non-skin side of the inner-layer sheet 23 (see FIGS. 2 and 3). The front outer-layer sheet 21 is laminated to the inner-layer sheet 23 mainly in the front portion 1A, and the back outer-layer sheet 22 is laminated to the inner-layer sheet 23 mainly in the back portion 1C. In a central portion between the front outer-layer sheet 21 and the back outer-layer sheet 22 in the longitudinal direction, a sheet member laminated to the inner-layer sheet 23 is not provided.

In the exterior member 20 of the diaper 1, portions of the side joining portions 2 that join the front portion 1A and the back portion 1C are portions to be grasped by hands and pulled upward when the diaper 1 is put on, as shown in FIG. 7B and the like. Accordingly, with a strength being secured by providing the exterior member 20 with a laminated structure including two or more layers in the vicinity of the two end portions in the longitudinal direction (the front portion 1A and the back portion 1C), tearing of the diaper 1 is likely to be suppressed. On the other hand, in the vicinity of a central portion (crotch portion 1B) in the longitudinal direction, which comes into contact with the wearer's crotch portion when the diaper 1 is worn, the exterior member 20 has a single-layer structure, and thus, the stiffness of the exterior member 20 is lower than that in the two end portions, thereby facilitating securing flexibility in the crotch portion. Accordingly, when the exterior member 20 is pulled upward, the shape of the convexly deformed absorbent main body 10 is less likely to collapse, and the absorbent main body 10 can more easily fit the wearer's crotch portion. Further, the leg portions LHC in the crotch portion of the exterior member 20 has sufficient flexibility, and thus even when the leg portions LHC come into contact with the wearer's groin region during wearing of the diaper 1, discomfort is less likely to be caused to the wearer.

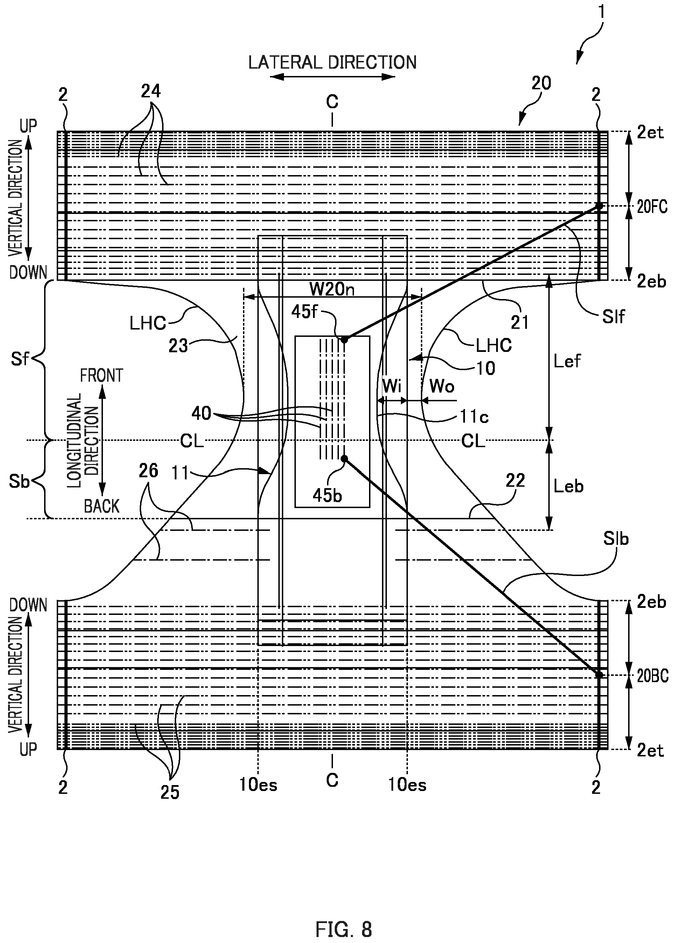

FIG. 8 is a plan view for illustrating a specific configuration of the diaper 1. As in FIG. 2, FIG. 8 illustrates the diaper 1 in an unfolded and stretched state. In FIG. 8, a portion located on the front side with respect to the central position CL in the longitudinal direction of the exterior member 20, in which the front outer-layer sheet 21 (second sheet) is not laminated to the inner-layer sheet 23 (first sheet), is defined as a front single-layer region Sf. Similarly, a portion located on the back side with respect to the central position CL in the longitudinal direction of the exterior member 20, in which the back outer-layer sheet 22 (second sheet) is not laminated to the inner-layer sheet 23 (first sheet), is defined as a back single-layer region Sb. In the diaper 1, the area of the front single-layer region Sf is greater than the area of the back single-layer region Sb (Sf > Sb). That is, in the exterior member 20 of the diaper 1, a single-layer portion is wider in a front-side region of the crotch portion than in a back-side region thereof, which facilitates securing of flexibility on the front side. Accordingly, when the diaper 1 is worn, the exterior member 20 becomes more easily deformed in a flexible manner in a region that comes into contact with the wearer's excretory opening (for example, a urethral opening, a vaginal opening, and/or the like), thereby making it less likely to cause discomfort to the wearer. Further, convex deformation of the absorbent main body 10 is less likely to be hindered in the vicinity of the excretory opening, thereby being able to improve the fit of the absorbent main body 10.

Further, in the exterior member 20 of the diaper 1, the waist elastic members 24 and 25 and the buttocks elastic members 26, configured to stretch and contract in the lateral direction, are provided between the inner-layer sheet 23 and the outer-layer sheet 21, 22 in the thickness direction. In the diaper 1, the waist elastic members 24 and 25 and the buttocks elastic members 26 are all arranged linearly along the lateral direction (see FIG. 8 and the like), and thus these elastic members 24 to 26 are hereinafter collectively referred to as "lateral elastic members." In the present embodiment, a shortest distance Lef from the central position CL to the lateral elastic members, on the front side with respect to the central position CL in the longitudinal direction, is greater than a shortest distance Leb from the central position CL to the lateral elastic members on the back side with respect to the central position CL in the longitudinal direction (Lef > Leb).

In FIG. 8, the distance Lef is the length from the central position CL to the front waist elastic member 24 that is located closest to the back side (the lowest side in the vertical direction) in the longitudinal direction. Similarly, the distance Leb is the length from the central position CL to the buttocks elastic member 26 that is located closest to the front side (the lowest side in the vertical direction) in the longitudinal direction. In the exterior member 20 of the diaper 1, a region in which the lateral elastic members (24 to 26) are provided has higher stiffness than other regions because at least two sheet members are laminated therein and because of the stiffness of the elastic members themselves. Accordingly, as the distance from the central position CL to a position at which the lateral elastic members are provided in the longitudinal direction increases, the area of a region having low stiffness can be increased. In the present embodiment, the distance Lef on the front side is greater than the distance Leb on the back side, and thus the area of the region having low stiffness is larger on the front side with respect to the central position CL in the longitudinal direction of the exterior member 20. Thus, flexibility of the exterior member 20 is secured over a wide area in the vicinity of the excretory opening located on the front side of the wearer's crotch portion. Further, convex deformation of the absorbent main body 10 is less likely to be hindered in the vicinity of the excretory opening, thereby being able to improve the fit thereof.

It should be noted that the exterior member 20 of the diaper 1 is not provided with an elastic member that is arranged so as to be inclined obliquely with respect to the longitudinal direction (the lateral direction). That is, the lateral elastic members (24 to 26) do not include an obliquely inclined portion. In conventional diapers, a leg elastic member inclined obliquely along each of the peripheral edges of the leg portions LHC may be provided, or a portion of the buttocks elastic member 26 may be curved along each of the leg portions LHC. In such a case, when the diaper is pulled upward as shown in FIG. 7B, an excessively large force is likely to act in an oblique direction due to the elastic force of the inclined elastic member, which may cause collapse of the convex deformation of the absorbent main body 10. In contrast, in the present embodiment, an obliquely inclined elastic member is not provided, and thus an excessively large force is less likely to act in an oblique direction when the exterior member 20 is pulled upward during wearing of the diaper 1, thereby suppressing collapse of the shape of the convexly deformed absorbent main body 10. Accordingly, deterioration of the fit is likely to be suppressed.

Further, in the diaper 1, the absorbent core 11 has, at a central portion in the longitudinal direction, a constricted portion 11c whose width in the lateral direction is smaller than the widths at the two end portions in the longitudinal direction (see FIG. 8 and the like). When a maximum distance between a peripheral edge of the constricted portion 11c and the corresponding outer end 10es of the absorbent main body 10 in the lateral direction is defined as Wi, and a minimum distance between a peripheral edge of each of the leg portions LHC and the corresponding outer end 10es of the absorbent main body 10 is defined as Wo, it is preferable that the distance Wi is greater than the distance Wo (Wi > Wo). By making the constriction of the absorbent core 11 in the lateral direction (that is, the distance Wi) as large as possible, the edge portions of the absorbent core 11 are less likely to interfere with the wearer's legs or groin region when the diaper 1 is worn, thereby making it less likely for the wearer to feel the hardness of the absorbent core 11. On the other hand, by making the length of a portion of the exterior member 20 protruding outward beyond the absorbent main body 10 in the lateral direction (that is, the distance Wo) as small as possible, the area of direct contact between the exterior member 20 and the wearer's skin (groin region) can be reduced, thereby making it less likely to cause discomfort to the wearer, when the diaper 1 is worn.

Further, in the diaper 1, a portion at which the width W20n of the exterior member 20 in the lateral direction is smallest is located on the front side with respect to the central position CL in the longitudinal direction. In other words, at a predetermined position located on the front side with respect to the central position CL in the longitudinal direction, the distance (W20n) between the pair of left and right leg portions LHC and LHC is smallest (see FIG. 8 and the like). The width W20n of the exterior member 20 is smallest at a portion of the crotch portion relatively on the front side, and thus when the wearer moves his or her leg(s) forward while wearing the diaper 1, for example, when assuming a sitting posture, walking, or the like, the edge portions of the exterior member 20 (that is, the peripheral edges of the leg portions LHC) are less likely to interfere with movements of the legs. This makes it possible to reduce discomfort in the crotch portion.

Furthermore, a portion at which the width W20n of the exterior member 20 in the lateral direction is smallest is located in a region overlapping the crotch elastic member 40 in the longitudinal direction (see FIG. 8 and the like). That is, a configuration is such that, on two lateral sides of a region in which the elastic force of the crotch elastic member 40 acts, the area of the exterior member 20 is minimized. By minimizing the area of the exterior member 20 on two sides of the crotch elastic member 40, resistance to convex deformation of the absorbent main body 10 caused in response to contraction of the crotch elastic member 40 is reduced, thereby allowing the absorbent main body 10 to be naturally deformed into a convex shape as shown in FIG. 5B. Accordingly, it is possible to further improve the fit of the absorbent main body 10 in the crotch portion.

FIG. 9 is a plan view illustrating an arrangement of the main-body joining portion 70 that joins the absorbent main body 10 and the exterior member 20. In the diaper 1, the absorbent main body 10 is joined to the exterior member 20 via the main-body joining portion 70 formed by an adhesive such as a hot-melt adhesive or the like. The main-body joining portion 70 is formed by applying the adhesive in a range indicated by hatching in FIG. 9 on the non-skin-side surface of the absorbent main body 10 or on the skin-side surface of the exterior member 20. In the present embodiment, the main-body joining portion 70 is provided over substantially the entire absorbent main body 10. That is, the absorbent main body 10 and the exterior member 20 are joined to each other on two lateral sides of the region overlapping the crotch elastic member 40 in the longitudinal direction. It should be noted that, in the main-body joining portion 70, the adhesive need not be uniformly applied over the entire region indicated by hatching in FIG. 9, and for example, a line-shaped adhesive may be applied in a spiral pattern or an Ω-shaped pattern instead.

If the absorbent main body 10 and the exterior member 20 are not joined to each other at portions located on two lateral sides of the region in which the elastic force of the crotch elastic member 40 acts, the two lateral end portions of the absorbent main body 10 may lift away from the exterior member 20 in the vicinity of the wearer's groin region when the diaper 1 is worn, which may cause discomfort or an unpleasant feeling to the wearer. In contrast, in the present embodiment, the absorbent main body 10 and the exterior member 20 are firmly joined and integrated with each other on the two lateral sides of the crotch elastic member 40, and thus lifting of the absorbent main body 10 and the like are less likely to occur. Accordingly, it is possible to reduce discomfort in the vicinity of the wearer's groin region when the diaper 1 is worn.

Further, in the present embodiment, the width of the main-body joining portion 70 in the lateral direction is substantially uniform over the entire length in the longitudinal direction. In FIG. 9, a width W60f of the main-body joining portion 70 at a front end portion in the longitudinal direction, a width W60b of the main-body joining portion 70 at a back end portion in the longitudinal direction, and a width W60c of the main-body joining portion 70 in a region overlapping the crotch elastic member 40 in the longitudinal direction are all equal to one another. With such a configuration, the absorbent main body 10 and the exterior member 20 are joined together entirely in a substantially uniform manner, and thus when the exterior member 20 is pulled upward during wearing of the diaper 1, stress concentration and the like are less likely to occur, thereby suppressing application of a localized force to the absorbent main body 10 via the exterior member 20. Accordingly, convex deformation of the absorbent main body 10 is less likely to be hindered, thereby facilitating improvement of the fit in the crotch portion.

Further, the absorbent main body 10 of the diaper 1 is not provided with elastic members configured go stretch and contract in the longitudinal direction at two lateral end portions thereof. Here, the phrase the "elastic members that are stretchable and contractable in the longitudinal direction at the two end lateral portions thereof" refers to elastic members having a function as "leg elastic members" configured to stretch and contract around the wearer's legs when worn, and refers to elastic members different from the leak-proof wall elastic members 61 of the present embodiment (see FIG. 4). In absorbent main bodies 10 employed in conventional underpants-shaped diapers, a technique of improving the fit around the wearer's legs by providing the "leg elastic members" at two lateral side portions of the absorbent main body 10 has been widely known. However, in the diaper 1 of the present embodiment, if elastic members corresponding to the "leg elastic members" were provided on the non-skin side with respect to the absorbent core 11 in the thickness direction and at the two lateral side portions of the absorbent main body 10 so as to stretch and contract along the longitudinal direction, convex deformation of the absorbent main body 10 might be hindered.

As described with reference to FIG. 5B, in the absorbent main body 10 according to the present embodiment, the absorbent core 11 easily undergoes convex deformation with the absorbent core 11 being pushed toward the skin side by the elastic force of the crotch elastic member 40 acting in the central region CR. In contrast, if elastic members corresponding to the leg elastic members were provided on the two lateral sides of the crotch elastic member 40, the absorbent core 11 would be pushed toward the skin side in the two side regions SR as well by the elastic force generated by the leg elastic members, which might make it difficult for convex deformation as shown in FIG. 5B to occur. Accordingly, in the present embodiment, by not providing the leg elastic members configured to stretch and contract along the longitudinal direction at the two lateral end portions, a contracting force along the longitudinal direction is less likely to act on the two side regions SR of the absorbent main body 10. This increases the effect of the elastic force imparted by the crotch elastic member 40, thereby facilitating convex deformation of the absorbent main body 10 in an intended manner.

Further, the exterior member 20 of the diaper 1 is not provided with elastic members that are stretchable and contractable in the lateral direction in a region overlapping the crotch elastic member 40 in the longitudinal direction. The exterior member 20 of the diaper 1 is provided with the above-described waist elastic members 24 and 25 and the buttocks elastic member 26 (lateral elastic members) as the elastic members that are stretchable and contractable in the lateral direction, however, none of these elastic members overlaps the crotch elastic member 40 in the longitudinal direction. If elastic members corresponding to the lateral elastic members were provided in the region overlapping the crotch elastic member 40 in the longitudinal direction, the elastic force of the crotch elastic member 40 in the longitudinal direction and the elastic force of the lateral elastic members in the lateral direction could interfere with each other, which may hinder stretching and contraction of the crotch elastic member 40. In contrast, in the present embodiment, lateral elastic members are not provided in the region overlapping the crotch elastic member 40 in the longitudinal direction, and thus stretching and contraction of the crotch elastic member 40 along the longitudinal direction are less likely to be hindered. Accordingly, it is possible to facilitate convex deformation of the absorbent main body 10 in an intended manner.

It is preferable that, when the diaper 1 is in an unfolded and stretched state, there is not a point of intersection existing between the peripheral edge of each of the leg portions LHC and a straight line connecting, by the shortest distance, an intermediate position in the longitudinal direction of each of the side joining portions 2 disposed on one side in the longitudinal direction and an end of the crotch elastic member 40 on the one side in the longitudinal direction. Similarly, it is preferable that there is not a point of intersection existing between the peripheral edge of each of the leg portions LHC and a straight line connecting, by the shortest distance, an intermediate position in the longitudinal direction of each of the side joining portions 2 disposed on the other side in the longitudinal direction and an end of the crotch elastic member 40 on the other side in the longitudinal direction.

In FIG. 8, a straight line Slf connecting a midpoint 20FC between a front end (upper end) 2et and a back end (lower end) 2eb of the side joining portion 2 located on the front side in the longitudinal direction and on the right side in the lateral direction and a point 45f located at the front end in the longitudinal direction and at the right end in the lateral direction of the crotch elastic member 40 does not intersect the peripheral edge of the leg portion LHC. Further, a straight line Slb connecting a midpoint 20BC between a front end (upper end) 2et and a back end (lower end) 2eb of the side joining portion 2 located on the back side in the longitudinal direction and on the right side in the lateral direction and a point 45b located at the back end in the longitudinal direction and at the right end in the lateral direction of the crotch elastic member 40 does not intersect the peripheral edge of the leg portion LHC.

When the diaper 1 is put on, an operation of grasping the two lateral side portions of the exterior member 20 and pulling it upward is performed, as shown in FIG. 7B. At such an event, assuming that the midpoints 20FC and 20BC of the side joining portions 2 are grasped, a force is easily transmitted along the straight lines (Slf, Slb) connecting the midpoints 20FC and 20BC and the crotch elastic member 40 by the shortest distances. If these straight lines intersect the peripheral edge of the leg portion LHC, the force acting along the straight line between the position at which the exterior member 20 is grasped (midpoint 20FC, 20BC) and the crotch elastic member 40 (45f, 45b) may be interrupted by the leg portion LHC, making it difficult for the force of pulling up the diaper 1 to be transmitted. That is, it may become difficult to efficiently pull up the absorbent main body 10 via the exterior member 20.

In contrast, in the diaper 1 according to the present embodiment, the straight lines (Slf and Slb) connecting the crotch elastic member 40 and the midpoints 20FC and 20BC by the shortest distances are not interrupted by the leg portions LHC, and thus the force of pulling up the diaper 1 can be easily transmitted along these straight lines. Accordingly, the absorbent main body 10 can be efficiently pulled upward via the exterior member 20. This enables the absorbent main body 10 to be more easily fitted to the wearer's crotch portion.

### Others

Embodiment(s) of the present disclosure described above is/are simply to facilitate understanding of the present disclosure and is/are not in any way to be construed as limiting the present disclosure. The present disclosure may variously be changed or altered without departing from its essential features and encompass equivalents thereof.

### REFERENCE SIGNS LIST

1 diaper (underpants-shaped disposable diaper, underpants-shaped absorbent article),
5 diaper (underpants-shaped disposable diaper of a comparative example),
1A front portion, 1B crotch portion, 1C back portion,
2 side joining portion,
10 absorbent main body, 10es end (outer end in the lateral direction),
11 absorbent core, 11c constricted portion,
12 top sheet, 13 back sheet, 15 intermediate sheet,
16 side sheet, 17 elastic-member cover sheet, 18 core-wrapping sheet,
19 leg-gather elastic member,
20 exterior member,
21 front outer-layer sheet, 22 back outer-layer sheet, 23 inner-layer sheet,
24 waist elastic member (front lateral elastic member),
25 waist elastic member (back lateral elastic member),
26 buttocks elastic member (lateral elastic member),
31 cover sheet, 32 cover sheet,
40 crotch elastic member,
41-45 crotch elastic member,
60 leak-proof wall portion,
61 leak-proof-wall elastic member,
70 main-body joining portion,
BH waist opening, LH leg opening,
LHC leg portion,
CL central position (longitudinal direction),
ER crotch elastic region,
CR central region, SR side regions,

## Claims

1. An underpants-shaped absorbent article having a longitudinal direction, a lateral direction, and a thickness direction that intersect each other in an unfolded and stretched state, the underpants-shaped absorbent article comprising:
an absorbent main body including an absorbent core;
an exterior member provided on a non-skin side with respect to the absorbent main body; and
a crotch elastic member,
the exterior member including, on two lateral sides thereof, a pair of leg portions obtained such that a width in the lateral direction at a central portion in the longitudinal direction is smaller than a width thereof in the lateral direction at two end portions in the longitudinal direction,
the crotch elastic member being provided on the non-skin side with respect to the absorbent core, the crotch elastic member being configured to stretch and contract along the longitudinal direction,
in the longitudinal direction, a length of the crotch elastic member on a front side with respect to a central position being greater than a length of the crotch elastic member on a back side with respect to the central position,
in the lateral direction, an entire peripheral edge of each of the leg portions being located on an outer side with respect to a corresponding outer end of the absorbent main body,
the exterior member does not include a leg elastic member extending across the central position in the longitudinal direction and configured to stretch and contract along the peripheral edge of each of the leg portions.

2. The underpants-shaped absorbent article according to claim 1, wherein
the exterior member includes
a first sheet extending continuously from one side to another side in the longitudinal direction, and
second sheets laminated to the first sheet at two end portions in the longitudinal direction, respectively, the second sheets not being laminated to the first sheet at a central portion in the longitudinal direction.

3. The underpants-shaped absorbent article according to claim 2, wherein
an area of a portion of the exterior member on a front side with respect to the central position in the longitudinal direction, the portion being where the second sheet is not laminated to the first sheet, is greater than an area of a portion of the exterior member on the back side with respect to the central position in the longitudinal direction, the portion being where the second sheet is not laminated to the first sheet.

4. The underpants-shaped absorbent article according to claim 2, wherein
in the exterior member, a lateral elastic member arranged linearly along the lateral direction is provided between the first sheet and each of the second sheets in the thickness direction, and
a shortest distance from the central position in the longitudinal direction to the lateral elastic member on the front side with respect to the central position is greater than a shortest distance from the central position to the lateral elastic member on the back side with respect to the central position.

5. The underpants-shaped absorbent article according to claim 4, wherein
the exterior member does not include an elastic member arranged obliquely with respect to the longitudinal direction.

6. The underpants-shaped absorbent article according to any one of claims 1 to 5, wherein
the absorbent core has, at a central portion in the longitudinal direction, a constricted portion having a width in the lateral direction smaller than a width at two end portions in the longitudinal direction, and
in the lateral direction, a maximum length between the outer end of the absorbent main body and a corresponding peripheral edge of the constricted portion is greater than a minimum length between the outer end of the absorbent main body and a corresponding peripheral edge of the leg portion.

7. The underpants-shaped absorbent article according to any one of claims 1 to 5, wherein
a portion of the exterior member having a smallest width in the lateral direction is located on the front side with respect to the central position in the longitudinal direction.

8. The underpants-shaped absorbent article according to claim 7, wherein
the portion of the exterior member having the smallest width in the lateral direction is located in a region overlapping the crotch elastic member in the longitudinal direction.

9. The underpants-shaped absorbent article according to any one of claims 1 to 5, wherein
a main-body joining portion that joins the absorbent main body and the exterior member is provided on two sides in the lateral direction of a region overlapping the crotch elastic member in the longitudinal direction.

10. The underpants-shaped absorbent article according to claim 9, wherein
a width of the main-body joining portion in the lateral direction in a region overlapping the crotch elastic member in the longitudinal direction is equal to a width of the main-body joining portion in the lateral direction at two end portions of the absorbent main body in the longitudinal direction.

11. The underpants-shaped absorbent article according to any one of claims 1 to 5, wherein
the absorbent main body does not include, at two end portions in the lateral direction, a leg elastic member configured to stretch and contract along the longitudinal direction.

12. The underpants-shaped absorbent article according to any one of claims 1 to 5, wherein
the exterior member does not include, in a region overlapping the crotch elastic member in the longitudinal direction, an elastic member configured to stretch and contract in the lateral direction.

13. The underpants-shaped absorbent article according to any one of claims 1 to 5, wherein
the exterior member includes, at two lateral end portions, a pair of side joining portions that join one-side end portions and another-side end portions in the longitudinal direction to each other, respectively, and
in the unfolded and stretched state,
a straight line connecting, by a shortest distance, a central position between a one-side end and another-side end in the longitudinal direction of each of the side joining portions provided on one side in the longitudinal direction and an end of the crotch elastic member on the one side in the longitudinal direction does not intersect a corresponding peripheral edge of the leg portion, and
a straight line connecting, by a shortest distance, a central position between a one-side end and another-side end in the longitudinal direction of each of the side joining portions provided on the other side in the longitudinal direction and an end of the crotch elastic member on the other side in the longitudinal direction does not intersect the corresponding peripheral edge of the leg portion.
